# EUROPEAN PATENT SPECIFICATION

(11) **EP 0 616 539 B1**
(45) Date of publication and mention of the grant of the patent: **04.03.1998**
(21) Application number: 92923894.7
(22) Date of filing: 20.11.1992
(51) Int. Cl.: A61M 1/00

(54) **SUCTION NIPPLE EVERSION DEVICE**
SAUGEINRICHTUNG ZUR AUSSTULPUNG VON BRUSTWARZEN
DISPOSITIF D'EVERSION DE SUCCION DES MAMELONS

(30) Priority: 10.12.1991 GB 9126230
(43) Date of publication of application: 28.09.1994
(73) Proprietor: McGEORGE, Douglas Donald, Chester, CH4 8JN (GB)
(72) Inventor: McGEORGE, Douglas Donald, Chester, CH4 8JN (GB)
(74) Representative: Cummings, Sean Patrick
(86) International application number: PCT/GB92/02159
(87) International publication number: WO 93/11806

(56) References cited:
- DE-C- 346 471
- GB-A- 1 210 746
- GB-A- 2 240 924

## Description

### BACKGROUND OF THE INVENTION

The present invention relates to a suction nipple eversion device for the correction of inverted nipples.

Inverted nipples are a common problem caused by short lactiferous ducts. Many operative procedures are available for their correction but all have limitations.

Remedial devices offering temporary nipple eversion to allow breast feeding are known. For example, GB 2,240,924A to Carter shows a nipple extractor device which is operated by pressing the flat rim of teat-shaped enclosure against the periphery of a retracted nipple and pressing down a handle assembly to collapse the side walls of the device and partly expel air from the enclosure. The handle is then pulled outwardly to return the enclosure to its normal configuration thereby creating suction inside the enclosure resulting in exposure of the nipple. The Carter device does not, however, offer a long-term solution to the problem of inverted nipples.

### SUMMARY OF THE INVENTION

It is therefore an object of the present invention to provide a simple device for correcting inverted nipples which obviates the need for operative intervention.

Accordingly, the present invention resides in a suction nipple eversion device comprising a nipple mould for receiving a nipple of the breast of a user of the device and a sealing flange for sealing the nipple mould against the nipple areolar complex of the breast under negative pressure produced upon withdrawal of air from the mould, wherein the device further comprises a valve in fluid communication with the mould to withdraw air from the mould and wherein the device in use is self-supporting upon the nipple areolar complex.

In use, the device according to the present invention operates by producing a suction effect on the inverted nipple,the negative pressure acting to draw out the inverted nipple into the mould. Since the device supports itself, individuals are free to move about during use. The device is worn for intervals over a period of time resulting in stretching of the lactiferous ducts and thereby providing a simple means for the correction of inverted nipples.

Preferably, the valve is provided with a syringe port and is attached to the nipple mould by means of a flexible tube. When the device is held against the nipple areolar complex of the breast air can be withdrawn through the valve by means of a syringe.

The nipple may be visualised during use through a transparent mould.

Removal is effected by simply lifting the device off the breast from the edge of the sealing flange so disrupting the seal and equalising the pressure.

### BRIEF DESCRIPTION OF THE DRAWINGS

The invention will now be described by way of example with reference to the accompanying drawings in which:
Figure 1 is a perspective view of a suction nipple eversion device.
Figure 2 is a perspective view of a suction nipple eversion device in use.
Figure 3 is a cross-sectional view of the device of Figures 1 and 2.

### DETAILED DESCRIPTION OF THE PREFERRED EMBODIMENTS

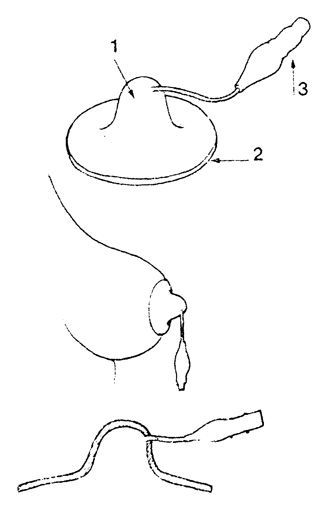

Figure 1 shows a suction nipple eversion device comprising a nipple mould (1) provided with a sealing Range (2). The mould (1) is provided with a valve connected to the mould (1) by means of a flexible tube. The valve (details of which are not shown) has a syringe port for receiving a syringe to extract air from the mould.

With the device held against the nipple areolar complex of the breast air can be withdrawn with a syringe. The negative pressure so created draws out the inverted nipple into the mould. The mould is transparent to permit the nipple to be visualised during use.

A perspective view of the device is shown at Figure 2 and a cross-sectional view shown at Figure 3.

## Claims

1. A suction nipple eversion device comprising a nipple mould (1) for receiving a nipple of the breast of a user of the device and a sealing flange (2) for sealing the nipple mould against the nipple areolar complex of the breast under negative pressure produced upon withdrawal of air from the mould, wherein the device further comprises a valve in fluid communication with the mould to withdraw air from the mould and wherein the device in use is self-supporting upon the nipple areolar complex.

2. A suction nipple eversion device according to claim 1 wherein the valve is provided with a syringe port.

3. A suction nipple eversion device according to claim 1 wherein the nipple mould (1) is transparent.

## Patentansprüche

1. Saugeinrichtung zur Ausstülpung von Brustwarzen, umfassend eine Brustwarzenform (1) zur Aufnahme einer Brustwarze der Brust einer Verwenderin der Einrichtung sowie einen Abdichtflansch (2) zum Abdichten der Brustwarzenform gegen den Brustwarzerhofbereich unter Unterdruck, der durch Abzug von Luft aus der Form erzeugt wird, wobei die Einrichtung darüberhinaus ein Ventil in Strömungsverbindung mit der Form umfaßt, um Luft aus der Form abzuziehen, und wobei die Einrichtung bei Gebrauch auf dem Brustwarzenhofbereich freitragend ist.

2. Saugeinrichtung nach Anspruch 1, wobei das Ventil mit einem Spritzenport versehen ist.

3. Saugeinrichtung nach Anspruch 1, wobei die Brustwarzenform (1) transparent ist.

## Revendications

1. Dispositif d'éversion de mamelon par aspiration comprenant un moule de mamelon (1) afin de recevoir le mamelon du sein d'une utilisatrice du dispositif et un rebord d'obturation (2) pour rendre étanche le moule de mamelon contre le complexe aréolaire du mamelon du sein sous l'effet de la pression négative produite lors de l'enlèvement de l'air du moule, où le dispositif comporte en outre une valve en communication fluidique avec le moule afin d'extraire l'air du moule et où le dispositif est autonome en utilisation sur le complexe aréolaire du mamelon.

2. Dispositif d'éversion de mamelon par aspiration selon la revendication 1, dans lequel la valve comporte un orifice pour seringue.

3. Dispositif d'éversion de mamelon par aspiration selon la revendication 1, dans lequel le moule de mamelon (1) est transparent.
